# EUROPEAN PATENT SPECIFICATION

(11) **EP 1 438 042 B1**
(45) Date of publication and mention of the grant of the patent: **06.09.2006**
(21) Application number: 02801965.1
(22) Date of filing: 24.10.2002
(51) Int. Cl.: A61K 31/4015, A61P 31/00, A61P 31/18, C07D 207/452

(54) **POLYMALEIMIDE COMPOUNDS AND THEIR MEDICAL USE**
POLYMALEINIMID-VERBINDUNGEN UND IHRE MEDIZINISCHE VERWENDUNG
COMPOSES DE POLYMALEIMIDES ET LEURS UTILISATION MEDICALE

(30) Priority: 25.10.2001 GB 0125658
(43) Date of publication of application: 21.07.2004
(73) Proprietor: SSL International Plc, Cambridge, Cambridgeshire CB4 0GZ (GB)
(72) Inventor: SOLANKI, Suren, SSL International plc, Cambridge, Cambridgeshire CB4 0GZ (GB); POTTER, William Duncan, Stapleford , Cambridge CB2 5FD (GB); ANDERSON, Lorraine, MRC Technology, Edinburgh EH4 2SP (GB)
(74) Representative: Crooks, Elizabeth Caroline
(86) International application number: PCT/GB2002/004798
(87) International publication number: WO 2003/035058

(56) References cited:
- EP-A- 0 345 356
- EP-A- 0 467 701
- WO-A-96/09406
- WO-A-97/27203
- GB-A- 1 116 173
- JP-A- 9 129 240
- US-A- 4 810 638
- US-A- 5 539 132
- US-A- 5 658 940
- CHERONIS J C ET AL: "A new class of bradykinin antagonists: synthesis and in vitro activity of bissuccinimidoalkane peptide dimers." JOURNAL OF MEDICINAL CHEMISTRY. UNITED STATES 1 MAY 1992, vol. 35, no. 9, 1 May 1992 (1992-05-01), pages 1563-1572, XP002246182 ISSN: 0022-2623
- EISSA, AMAL A. H.: "Synthesis and HIV-1 activity of some novel 2,2'-diphenylamine derivatives" BULLETIN OF THE FACULTY OF PHARMACY (CAIRO UNIVERSITY), vol. 36, no. 3, 1998, pages 99-105, XP001164121
- DATABASE CA [Online] CHEMICAL ABSTRACTS SERVICE, COLUMBUS, OHIO, US; IBRAHIM, MOHAMED KAMAL: "[1,1'-Bipyrrolidine]-2,2',5,5'-tetrone Synthesis and biological activity of certain bi - imides" retrieved from STN Database accession no. 124:289228 HCA XP002251456 & AL-AZHAR JOURNAL OF PHARMACEUTICAL SCIENCES (1994), 13, 74-83 ,
- NAGAMURTHI G ET AL: "GRAMICIDIA-S: STRUCTURE-ACTIVITY RELATIONSHIP" JOURNAL OF BIOSCIENCES, INDIAN ACADEMY OF SCIENCES, XX, vol. 7, no. 3/4, June 1985 (1985-06), pages 323-329, XP001150332 ISSN: 0250-5991
- PATEL S V ET AL: "ARYL HYDRAZINE DERIVATIVES OF BISMALEIMIDES" ORIENTAL JOURNAL OF CHEMISTRY, IQBAL, BHOPAL,, IN, vol. 17, no. 2, 2001, pages 299-302, XP001150354 ISSN: 0970-020X
- GARBER G E ET AL: "CHARACTERIZATION AND PURIFICATION OF EXTRACELLULAR PROTEASES OF TRICHOMONAS-VAGINALIS" CANADIAN JOURNAL OF MICROBIOLOGY, vol. 35, no. 10, 1989, pages 903-909, XP008002969 ISSN: 0008-4166
- FILHO, VALDIR CECHINEL ET AL: "Antibacterial activity of N-phenylmaleimides, N-phenylsuccinimides and related compounds. Structure-activity relationships" FARMACO (1994), 49(10), 675-7, XP001068205
- PATENT ABSTRACTS OF JAPAN vol. 016, no. 375 (C-0973), 12 August 1992 (1992-08-12) & JP 04 120004 A (ICHIKAWA GOUSEI KAGAKU KK), 21 April 1992 (1992-04-21)
- ANDRUSZKIEWICZ R ET AL: "STRUCTURAL DETERMINANTS OF INHIBITORY ACTIVITY OF N3-(4-METHOXYFUMAROYL)-L-2,3-DIAMINOPROPAN OIC ACID TOWARDS GLUCOSAMINE-6-PHOSPHATE SYNTHASE" POLISH JOURNAL OF CHEMISTRY, POLISH CHEMICAL SOCIETY, XX, vol. 67, no. 4, April 1993 (1993-04), pages 673-683, XP000964579
- PATENT ABSTRACTS OF JAPAN vol. 017, no. 594 (C-1126), 29 October 1993 (1993-10-29) & JP 05 178825 A (YAMANOUCHI PHARMACEUT CO LTD), 20 July 1993 (1993-07-20)
- SIRCAR SUCHETA ET AL: "Inhibition of adenovirus infection with protease inhibitors." ANTIVIRAL RESEARCH, vol. 30, no. 2-3, 1996, pages 147-153, XP001076687 ISSN: 0166-3542

## Description

The present invention relates to the use of poly-maleimide derivatives as microbicidal agents. In particular it relates to the use of poly-maleimide derivatives, preferably bis-maleimide compounds in the treatment of microbial infections of the reproductive tract, such as bacterial, fungal, protozoal and viral infections.

Despite efforts to reduce the levels of sexually transmitted infections (STTs) for example by providing relevant information and education, and by promoting the use of barrier contraceptives, such as condoms, the level of such infections worldwide is increasing. There is therefore a continuing requirement for safe and effective therapeutic agents for the prophylaxis and treatment of STI's, in particular, agents which can readily be administered or applied at the time of sexual contact. Maleimides are a well known class of chemical compounds.

Maleimide derivatives are known to have a wide variety of utilities, and find application in the biological and medicinal fields, as well as in general industrial use, for example in the preparation of polymers and inks.

Thus for example, USP 3,129,225 (United States Vitamin and Pharmaceutical Corporation) describes maleimido betaines said to have antimicrobial and antiparasitic activity as well as pharmacological activity, in particular the reduction of serum cholesterol.

German ALS 1,140,192 (United States Rubber Company) describes a novel bismaleimide with a CH₂-O-CH₂ bridge, and its use in vulcanisation, cross-linking of polymers and as fungicide for tomatoes.

USP 4,876,358 (Texaco Inc.) describes novel bismaleimides having a (poly)oxyethylene bridge, for use in preparing polymers.

USP 5,552,396 and WO 97/19080 (Eli Lilly) describe classes of N,N-bridged bisindolylmaleimides which are said to be inhibitors of protein kinase C, and to be useful for the treatment of diabetes, ischemia, inflammation, cns disorders, cardiovascular disease, dermatological disease and cancer.

USP 5,380,764 (Goedecke AG) describes bis-indolemaleinimides, said to be protein kinase C inhibitors useful for treating diseases of heart or blood vessels, inflammatory processes, allergies, cancer and degenerative damage of cns, diseases of the immune system and viral diseases, including diseases caused by retroviruses HTLV -I, -II, -III.

WO 96/09406 (The Government of the United States of America, represented by the Secretary of the Department of Health and Human Services) describes the use of disulfides, maleimides and a variety of other electron acceptor compounds in inactivating HIV-1 and other retroviruses.

Igarashi et al., J. Industrial Microbiology, 6 (1990) pp223-225, report that various N-alkylmaleimides show antibacterial and antifungal activity.

We have found that poly-maleimide derivatives of formula (I) inhibit the infectivity of the human HIV-1 virus. Such maleimides of formula (I) are useful in the prophylaxis and/or treatment of a variety of sexually transmitted infections and other infections which affect the reproductive system, including reproductive organs and reproductive tract.

In a first aspect, there the present invention provides the use of a polymaleimide derivative of formula (I) in the manufacture of a medicament for the prophylaxis and/or treatment of microbial infections, in particular, sexually transmitted infections and other infections which affect the reproductive system.

Microbial STIs and other infections which affect the reproductive system include bacterial, fungal, protozoal and viral infections.

Bacterial infections include, chancroid, chlamydia, gonorrhoea, syphylis and bacterial vaginosis. Causitive agents of such infections include *H. ducreyi, C. trachomatis, N. gonorrhoea, T. pallidum* and anaerobic bacteria such as *G. vaginalis.*

Fungal infections include candidiasis (commonly known as thrush) which is caused by *C. albicans.*

Protozoal infections include trichomoniasis, caused by *T. vaginalis.*

Viral STI's include those caused by a virus selected from HIV (AIDS) HSV (herpes simplex) HPV(human papilloma virus) and HBV (hepatitis B).

The present invention therefore provides the use of a poly-maleimide compound of formula (I) in the manufacture of a medicament for the prophylaxis and/or treatment of a bacterial, fungal or protozoal infection of the reproductive system. The term poly-maleimide compound or derivative includes all derivatives, salts and substituted maleimides, including optionally substituted bis-maleimides and poly-maleimides. It does not relate to mono-maleimide compounds.

The present invention also provides the use of a maleimide derivative of formula (I) in the manufacture of a medicament for the prophylaxis and/or treatment of a viral infection.

The maleimide compound may preferably be represented by the general formula (I): 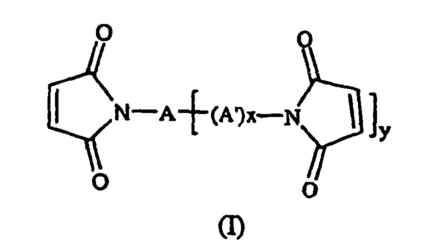 wherein
A is represented by (R¹)ₙ-(R²)ₚ-(R³)_{q}, wherein each of R¹, R² and R³ is independently selected from:
an acyclic aliphatic group having from 1-20 carbon atoms;
a polyalkylene glycol group having an average molecular weight in the range 100 to 1000;
a cyclic aliphatic group having from 4-20 carbon atoms;
a monocyclic or polycyclic aromatic hydrocarbon group having from 6-18 ring carbon atoms;
a non-aromatic heterocyclic group having from 3 to 8 ring atoms, or
a monocyclic or polycyclic aromatic heterocyclic group having from 5 to 20 ring atoms;
and n, p and q each represent 0 or 1 such that the sum of n+p+q = 1-3;
or A substituted by one or more groups independently selected from: 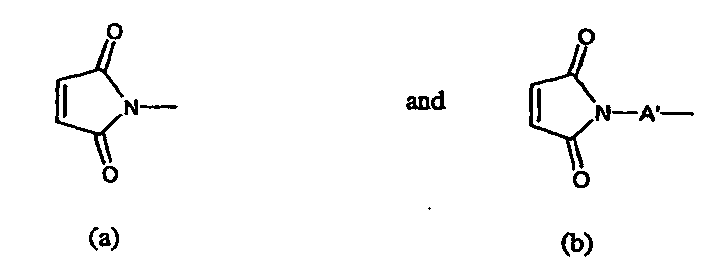
wherein A', which may be the same as or different from A, represents a group (R¹)ₙ-(R²)ₚ-(R³)_{q}, wherein R¹, R², R³, n, p and q are as defined above and wherein X is 0 or 1 and y is 1 to 6;
or a salt, ester or other physiologically equivalent derivative thereof.

In compounds of formula (I) it is preferred that the total number of atoms in the moieties A and A' does not exceed 50.

In the compounds of formula (I) above, an acyclic aliphatic group or moiety may be branched or unbranched alkyl, which alkyl group may be optionally interrupted by one or more oxygen atoms; alkenyl; or alkynyl; any of which may be optionally substituted by one or more substituents selected from the group consisting of halogen, cyano, nitro, -C(O)R⁴, -CO₂R⁴, -OR⁴, -SR⁴-SOR⁴, -SO₂R⁴, -NR⁴R⁵, -C(O)NR⁴R⁵; wherein R⁴ and R⁵ independently represent hydrogen, C₁₋₈alkyl, aryl or arylC₁₋₈alkyl.

For the purposes of the present invention, alkyl groups may have from 1 to 20 carbon atoms, preferably from 1 to 15 carbon atoms, more preferably from 1 to 10 carbon atoms. Alkenyl groups may have from 2 to 20 carbon atoms, preferably from 2 to 15 carbon atoms, more preferably from 2 to 10 carbon atoms. Alkynyl groups may have from 2 to 20 carbon atoms, preferably from 2 to 15 carbon atoms, more preferably from 2 to 10 carbon atoms.

A polyalkyleneglycol group may be a polyethylene glycol (PEG) or a polypropylene glycol which may be represented by the formulae -(OCH₂CH₂)ₘOH, -(OCH(CH₃)CH₂)ₘOH or -(OCH₂CH₂CH₂)ₘOH, wherein m is in the range 4 to 20. It will be appreciated that polyalkylene glycols are generally a mixture of chain lengths and are therefore conventionally described in terms of average molecular weight and average values of m. Thus for example PEG 200 has molecular weight in the range 190-210 and an average value of m=4 (The Merck Index, Eleventh edition).

Aryl is phenyl optionally substituted by one or more substituents selected from the group consisting of halogen, cyano, nitro, -C(O)R⁶, -CO₂R⁶, -OR⁶, -SR⁶-SOR⁶, -SO₂R⁶, -NR⁶R⁷, -C(O)NR⁶R⁷ wherein R⁶ and R⁷ independently represent hydrogen, or C₁₋₈alkyl.

A cyclic aliphatic group or moiety may be cycloalkyl or cycloalkenyl, either of which may be optionally substituted by one or more substituents selected from the group consisting of halogen, cyano, nitro, -C(O)R⁴, -CO₂R⁴, -OR⁴, -SR⁴ -SOR⁴, -SO₂R⁴, -NR⁴R⁵, -C(O)NR⁴R⁵; wherein R⁴ and R⁵ are as defined above.

Cycloalkyl groups of the present invention may have from 3 to 20 carbon atoms, preferably from 3 to 10 carbon atoms, more preferably from 3 to 6 carbon atoms. Cycloalkenyl groups of the present invention may have from 3 to 20 carbon atoms, preferably from 3 to 10 carbon atoms, more preferably from 3 to 6 carbon atoms.

Aromatic hydrocarbon groups and moieties include for example optionally substituted unsaturated monocyclic, fused bicyclic or tricyclic rings of up to 18 carbon atoms, such as phenyl and naphthyl, and partially saturated bicyclic or tricyclic rings such as tetrahydro-naphthyl. Aromatic groups also include linked aromatic rings, such as a biphenyl group. The aromatic rings may also be linked by heteroatoms, e.g. O, S or NR⁴. Examples of substituents which may be present on an aromatic group or moiety include one or more of halogen, alkyl, haloalkyl, cyano, nitro, -C(O)R⁴, -CO₂R⁴, -OR⁴, -SR⁴ -SOR⁴, -SO₂R⁴, -NR⁴R⁵, or -C(O)NR⁴R⁵. Substituents on the aromatic ring are preferably in the ortho or para position with respect to the maleimido group.

An aromatic heterocyclic group or moiety may be unsaturated or partially saturated, for example an optionally substituted 5- or 6-membered heterocyclic aromatic ring which may contain from 1 to 4 heteroatoms selected from O, N and S. The heterocyclic ring may optionally be fused to one or more phenyl rings. Examples of heteroaryl groups thus include furyl, thienyl, pyrrolyl, oxazolyl, oxazinyl, thiazolyl, imidazolyl, oxadiazolyl, thiadiazolyl, pyridyl, triazolyl, triazinyl, pyridazyl, pyrimidinyl, pyrazolyl, indolyl, indazolyl, benzofuranyl, benzothienyl, benzimidazolyl,-benzoxazolyl, benzoxazinyl, quinoxalinyl, quinolinyl, quinazolinyl, cinnolinyl, benzothiazolyl, pyridopyrrolyl Examples of substituents which may be present on a heterocyclic aromatic group include one or more of halogen, oxo,, nitro, cyano, alkyl, haloalkyl, -C(O)R⁴, -CO₂R⁴, -OR⁴, -SR⁴-SOR⁴, -SO₂R⁴,-NR⁴R⁵, or -C(O)NR⁴R⁴, wherein R⁴ and R⁵ are as defined above. Partially saturated derivatives of the aforementioned heteroaryl groups include 3-pyrroline and 1,2-dihydroquinoline.

Non-aromatic heterocyclic groups include pyrrolidinyl tetrahydrofuryl, tetrahydrothienyl and piperidinyl.

In the compounds of formula (I) R¹ preferably represents an aromatic hydrocarbon group, e.g. phenyl or naphthyl; or an aromatic heterocyclic group e.g.thienyl or acridine. Optional substituents present on the phenyl group are preferably in the ortho or para position (relative to maleimido) and may be for example selected from amino, halogen, eg chlorine, C₁₋₄alkyl, nitro, phenoxy and phenylamino.

When A represents phenyl the maleimido moieties are preferably in the para position relative to each other. When A represents biphenyl the bonds between the maleimido and phenyl groups and the bond between the two phenyl groups are are also preferably in the para positions.

A preferred class of compounds for use according to the invention is that of bismaleimide derivatives represented by formula (II): 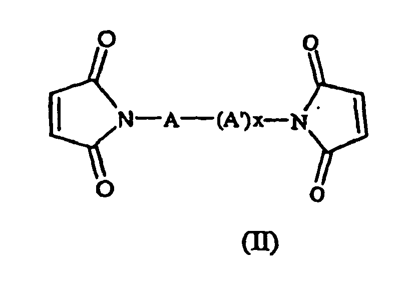 wherein A, A¹ and x are as defined above.

In the compounds of formula (II) x preferably represents 0 and A is as defined above.
In the group A, R¹, R², R³, n, p and q are preferably selected such that A represents a C₁₋₈alkyl group, optionally interrupted by an oxygen atom;
a polyalkylene glycol group having an average molecular weight in the range 100 to 1000; or
a phenyl group; or
n and q are preferably each 1, p represents 0 or 1,R¹ and R³ preferably each represent phenyl and, when p is 1, R² preferably represents a C₁₋₈alkyl group, optionally interrupted by an oxygen atom; or
a polyalkylene glycol group having an average molecular weight in the range 100 to 1000.

When A represents phenyl, the maleimido moieties are preferably in the para position relative to each other. When A represents biphenyl, the bonds between the maleimido and phenyl groups and the bond between the two phenyl groups are are also preferably in the para positions.

Maleimide derivatives for use according to the present invention are commercially available or can be prepared according to methods well known in the art.

A maleimide derivative for use according to the present invention may be administered as the sole active ingredient or may be administered in combination with one or more other maleimide derivatives as defined herein and/or in combination with one or more other therapeutic agents. In this context 'in combination' means that the compounds may be administered simultaneously or sequentially, and there may be an interval of time between administration of the two or more compounds. Other therapeutic agents which may be administered in combination with one or more maleimide derivatives include antibacterial, antifungal and antiviral agents.

In a second aspect, the present invention provides the use of a compound of formula (I) and preferably the use of a compound of formula (II) for the prophylaxis and/or treatment of retroviral infections of the reproductive tract, in particular, HIV infection.

The third aspect of the invention provides a pharmaceutical formulation comprising a compound as defined for the first aspect and a pharmaceutically acceptable excipient

For use in the treatment of STIs the compounds may be formulated for administration to the reproductive organs or reproductive tract (vaginal, cervical or uterine) or for rectal, buccal or oral administration. For administration to the reproductive organs or reproductive tract, or rectal administration the compounds may be formulated using conventional methods known in the art. Thus for example they may be formulated as creams, gels including thermoreversible gels, lubricants, pessaries, foaming tablets, aerosol foams, sprays, douches or films. The compounds may be incorporated into sustained release formulations, e.g. by micro-encapsulation, formulation with a bio-adhesive material, such as poly-carbophil, or other conventional means. Gels, creams and pessaries may, according to circumstance, be administered via an appropriate applicator. The formulations, in particular gels, creams and pessaries may be packaged with an appropriate applicator to facilitate administration.

Compounds and compositions for use according to the present invention may also be incorporated into contraceptive devices. Thus for example they may be incorporated into vaginal, cervical or uterine devices such as vaginal sponges or rings, and diaphragms or used to lubricate and/or coat condoms.

For buccal and oral administration the compounds may be formulated as lozenges, chewable tablets, wafers, mouthwashes and the like.

For use according to the present invention, a maleimide derivative may be administered at a dosage in the range 10-1000mg, e.g. 30 to 1000mg.

A fourth aspect of the invention provides a compound of formula (V) 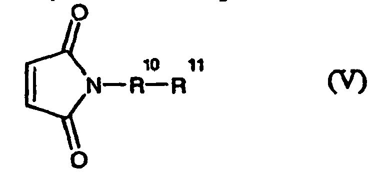 wherein R¹¹ is 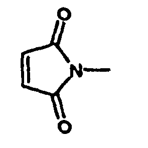 and R¹⁰ is C₂₋₁₀ alkyl, preferably wherein the alkyl group has 2, 3, 4, or 5 carbon atoms, or a group R²⁰-R²¹-R²²
wherein R²⁰ is aryl, preferably phenyl;
R²¹ is -(OCH₂CH₂)ₙ- wherein n represents 3 to 15, preferably n is one of 4, 5, 6, 7, 8, 9, 10, 11,12 and 13; and R²³ is C₁₋₁₀ alkyl, or R₂₁ is - (OCH₂-CH₂)₁₅
and R²² is a group O-R²³ wherein R²³ is aryl.

Preferred compounds of the fourth aspect are set out in the table below.

| **Compound number** | **Structure** |
|---|---|
| 1 | 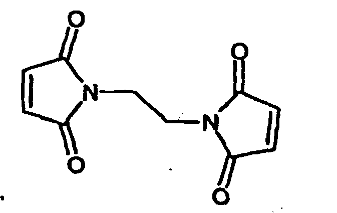 |
| 2 | 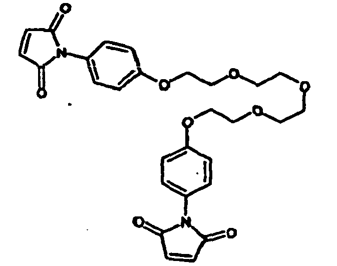 |

The fourth aspect of the invention also relates to salts, esters, amides or prodrugs of the compounds of formula V.

The compounds of the fourth aspect can be synthesised according to methods well known in the art. In particular, PEG of the appropriate chain length is initially converted to the aminoaryl derivative using conventional chemical synthesis. One example of such a chemical synthesis includes the coupling of PEG with p-fluoronitrobenzene in the presence of sodium hydride and DMF to produce a nitro-substituted aryl-PEG moiety. The nitro-subsituted aryl-PEG moiety is then reduced with a reducing agent such as palladium/carbon to form the required amine compound. The amine compound can then be converted into the maleimide compound of formula V by a number of methods known in the art. One example of such a method includes reacting the amine with maleic anhydride.

The fifth aspect of the invention provides a compound of formula V as defined in the fourth aspect of the invention for use in medicine. The compound of formula V is preferably provided as for the prevention or treatment of microbial infections which effect the reproductive tract. The compounds can be used to prevent or treat sexually transmitted infections and other infections which affect the reproductive tract.

All preferred features of different aspects of the invention apply to each other *mutatis mutanadis.*

The invention will be further illustrated by the following examples:

### Biological testing

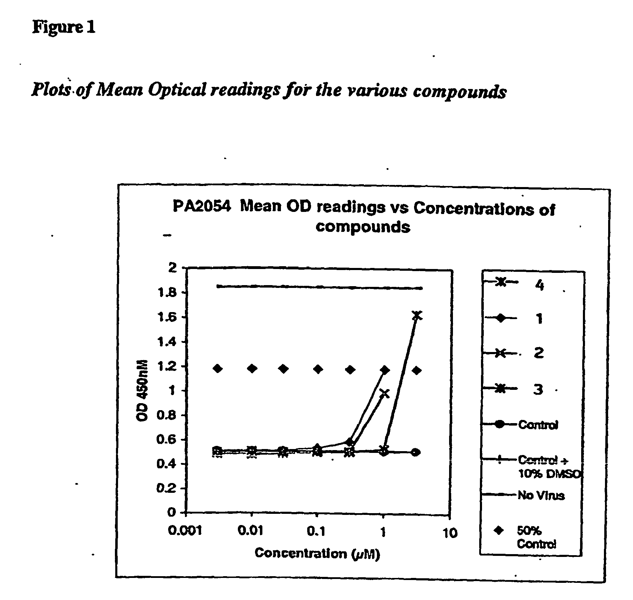

Figure 1 indicate optical density measurements versus various concentrations of the compounds of the invention.

### METHODS

### Chlamydia Assay

Purified Chlamydia trachomatis (250µl) was added to 250 µl of each compound concentration and the mixtures incubated at room temperature for 5 minutes. The tubes were then centrifuged (15000 rpm, 15 minutes), the supernatant decanted and the pellet washed serum free MEM (with 0.5mls). This step was repeated and the resultant pellet resuspended MEM (in 0.5ml) and inoculated into a confluent shell vial (coverslip) cultures of McCoy cells. The cultures were centrifuged (3000 rpm, 1 hour at 37°C) and incubated for a further 2 hours prior to being changed onto MEM with 10% foetal calf serum, I µg/ml cycloheximide, 0.5% added glucose, vancomycin, streptomycin and amphoteracin B (Fungizone). The cultures were incubated for 68 hours, fixed in methanol and stained with Lugol's iodine. 3 replicates of each positive control (with and without DMSO) were included, as well as 2 uninoculated cell controls. Each coverslip culture was examined using low power (10X) objective to ensure that inclusions were evenly distributed and 5 high power (X40) fields were selected at random from different areas of the coverslip. Iodine stained inclusions were counted. Where no inclusions were seen on high power, the culture was re-examined under low power.

### Anti-Viral Assay

HIV was attached to the surface of 96-well cell culture plates using 100µl of 50µg/ml poly L-lysine hydrobromide (PLL) of molecular weight >300K for 1h at RT. The outer wells were left uncoated and filled with 250µl of PBS to counteract the effects of evaporation. The coated wells were washed twice with PBS and 25µl of RPMI 1640 medium containing 5 x 10³ TCID₅₀ of HIV-1_{RF} was added and incubated for 1h at RT. The wells were then washed twice with PBS and 25µl of varying concentrations of the test compounds made up with serum free medium (containing a constant 10% DMSO) were added to the wells in quadruplicate and the plates incubated at 37°C for 10 minutes. The wells were again washed twice with PBS and 5 x 10⁴ C8166 cells in a volume of 300µl distributed to each well and the plates incubated for 5 and 6 days respectively. On days 5 and 6 the cells were assessed microscopically for the presence of cytopathic effect (syncytium formation) and recorded. In order to quantify the cytopathic effect, XTT which is metabolised by viable but not dead cells, was added and the absorbance read at 450nm after 3-4hr incubation at 37°C.

### Test Compounds

The following compounds of the invention were tested as set out in the above methods.

| **Compound number** | **Structure** |
|---|---|
| 1 | 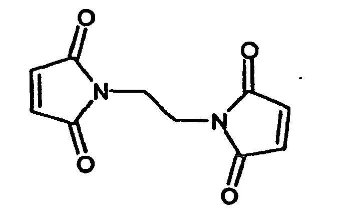 |
| 2 | 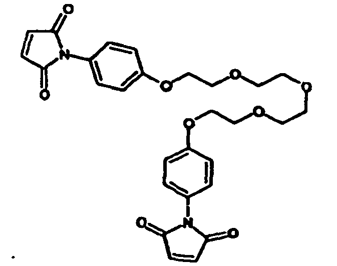 |

The comparative compounds, ethyl maleimide and PEG(5)PhMA were also used in the above methods.

| | |
|---|---|
| Comparative compound 3 (PEG(5)PhMA) | 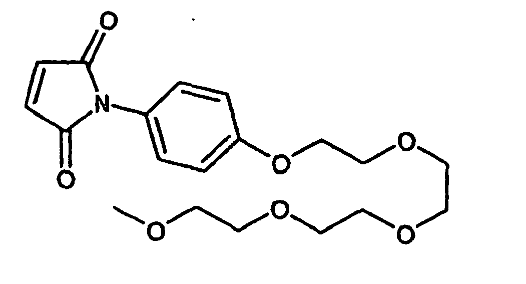 |
| Comparative compound 4 (Ethyl maleimide) | 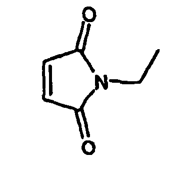 |

### RESULTS

### Chlamydia Assay: Table 1

There is no apparent difference between the number of inclusions found in McCoy cells exposed to chlamydia transiently treated with either media (DMEM) or vehicle treated chlamydia (DMSO, 10% final) suggesting that this concentration of DMSO does not compromise the ability of chlamydia to infect McCoy cells. Transient exposure of chlamydia to compounds 4, 1, 3 and 2 resulted in a dose-dependent inhibition of the infection of McCoy cells with chlamydia. Ethyl maleimide (4) is less active than its bis maleimide equivalent, ethyl bis-maleimide (1). Similarly PEG(5) phenyl maleimide (3) is less active than its PEG bis (phenyl maleimide) equivalent, PEG(5) bis phenyl bis maleimide (2).

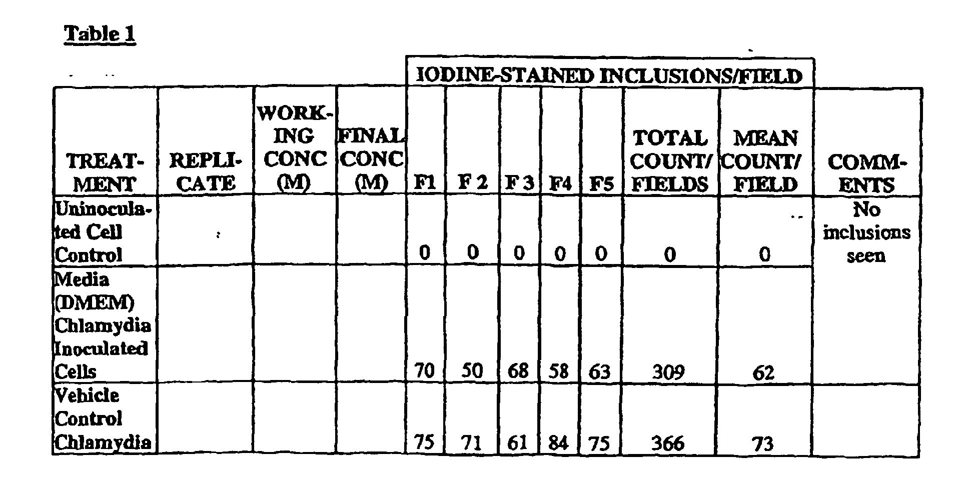

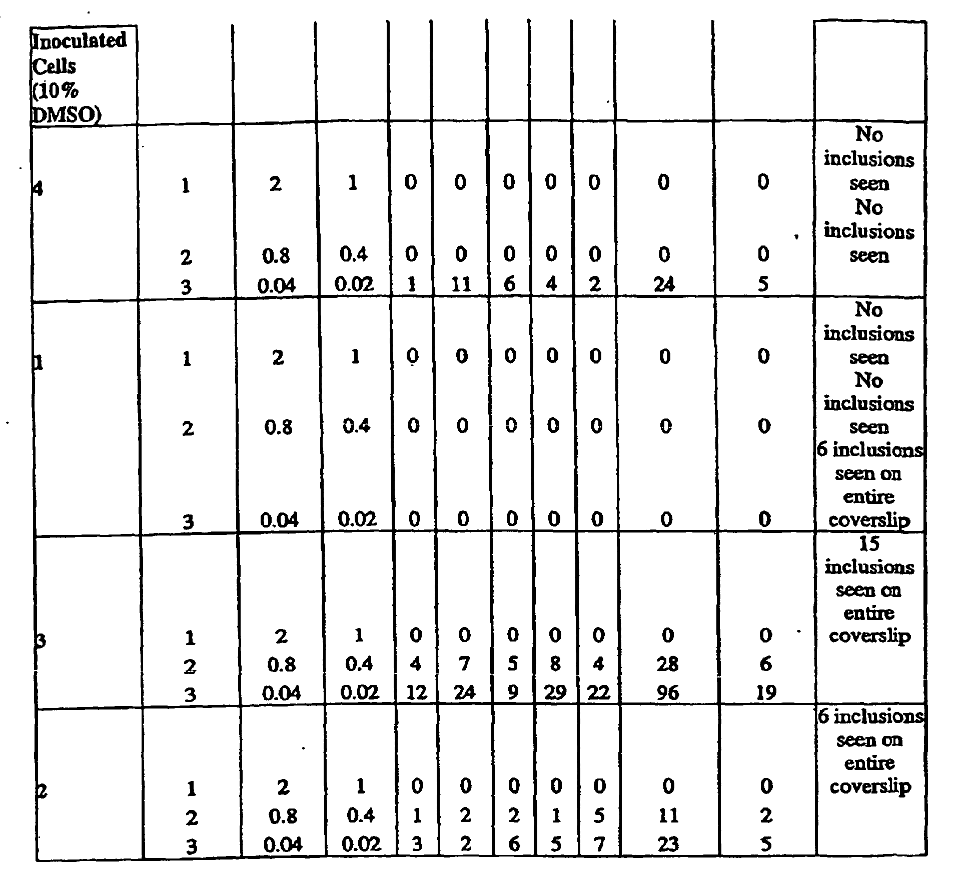

Table 1. Effect of Transient Treatment with maleimides on Chlamydia Infection of McCoy Cell *In Vitro.* Chlamydia was pre-treated with each of the indicated maleimides for 5 minutes and the compound removed by centrifugation. McCoy cells were then exposed to the compound pre-treated chlamydia for 68 hours and inclusions counted following staining of cells with Lugols iodine.

### Anti-Viral Assay

### RESULTS

An assessment of viral cytopathic effect was made microscopically on days 5 and 6, but the end point of the experiment was on day 6 when the level of virus infection (as determined by cell viability) was quantified by the use of XTT. There is a good correlation between the microscopic scores for syncytium formation and the OD values obtained by XTT on day 6 and the presence of 10% DMSO in the medium used to treat the virus did not appear to make any difference to the results.

The results of this experiment indicate that the most potent virucidal compound of the series was compound, 1 (IC₅₀ = 1.0mM), collowed in order of activity by 2 (almost 50% inhibition at ImM), 4 (IC₅₀ = 1.9mM) and 3 which showed no virucidal activity at concentrations up to 1.0mM.

**Table 2**

| **Compound** | **Conc (mM)** | **Syncytia score day 5 (quadruplicate wells)** | | | | **Syncytia score day 6 (quadruplicate wells)** | | | |
|---|---|---|---|---|---|---|---|---|---|
| | | | | | | | | | |
| **1** | 1 | ++ | ++ | 0 | 0 | ++ | ++/+++ | 0 | 0 |
| | 0.3 | +++ | +++ | +++ | +++ | +++ | +++ | +++ | +++ |
| | 0.1 | ++++ | ++++ | ++++ | ++++ | ++++ | ++++ | ++++ | ++++ |
| | 0.03 | ++++ | ++++ | ++++ | ++++ | ++++ | ++++ | ++++ | ++++ |
| | 0.01 | ++++ | ++++ | ++++ | ++++ | ++++ | ++++ | ++++ | ++++ |
| | 0.003 | ++++ | ++++ | ++++ | | ++++ | ++++ | ++++ | ++++ |
| | | | | | | | | | |
| **2** | 1 | +++ | ++ | ++ | 0 | +++ | +++ | ++ | 0 |
| | 0.3 | +++ | +++ | +++ | +++ | ++++ | ++++ | ++++ | ++++ |
| | 0.1 | ++++ | ++++ | +++ | ++++ | ++++ | ++++ | ++++ | ++++ |
| | 0.03 | ++++ | ++++ | ++++ | ++++ | ++++ | ++++ | ++++ | ++++ |
| | 0.01 | ++++ | ++++ | ++++ | ++++ | ++++ | ++++ | ++++ | ++++ |
| | 0.003 | ++++ | ++++ | ++++ | ++++ | ++++ | ++++ | ++++ | ++++ |
| | | | | | | | | | |
| **3** | 1 | +++ | +++ | +++ | +++ | +++ | +++ | +++ | +++ |
| | 0.3 | +++ | +++ | +++ | +++ | ++++ | ++++ | ++++ | ++++ |
| | 0.1 | +++ | +++ | +++ | +++ | ++++ | ++++ | ++++ | ++++ |
| | 0.03 | +++ | +++ | +++ | +++ | ++++ | ++++ | ++++ | ++++ |
| | 0.01 | +++ | +++ | +++ | +++ | ++++ | ++++ | ++++ | ++++ |
| | 0.003 | ++++ | ++++ | ++++ | ++++ | ++++ | ++++ | ++++ | ++++ |
| | | | | | | | | | |
| **4** | 3 | 0 | 0 | 0 | 0 | 0 | 0 | 0 | +/- |
| | 1 | +++ | +++ +++ | | +++ | ++ | ++ | +++ | +++ |
| | 0.3 | ++++ | ++++ | ++++ | ++++ | ++++ | ++++ | ++++ | ++++ |
| | 0.1 | ++++ | ++++ | ++++ | ++++ | ++++ | ++++ | ++++ | ++++ |
| | 0.03 | ++++ | ++++ | ++++ | ++++ | ++++ | ++++ | ++++ | ++++ |
| | 0.01 | ++++ | ++++ | ++++ | ++++ | ++++ | ++++ | ++++ | ++++ |
| | | | | | | | | | |
| **Virus Control + DMSO** | | ++++ | ++++ | ++++ | ++++ | ++++ | ++++ | ++++ | ++++ |
| | | | | | | | | | |
| **Virus Control + Medium only** | | ++++ | ++++ | ++++ | ++++ | ++++ | ++++ | ++++ | ++++ |
| | | | | | | | | | |
| **No virus Control** | | 0 | 0 | 0 | 0 | 0 | 0 | 0 | 0 |

| | | | | | | | | | |
|---|---|---|---|---|---|---|---|---|---|
| **Key:** | | | | | | | | | |
| 0= No syncytia; += <10% syncytia; ++ =10-25% syncytia; +++= 25-50% syncytia; ++++ = 50-100% syncytia. | | | | | | | | | |

**Table 3**

| ***Mean Optical Readings at 450nm*** | | | | | | | | |
|---|---|---|---|---|---|---|---|---|
| **Conc mM 3** | **4** 1.6305 | **1** nd | **2** nd | **3** nd | **Controls** | | | |
| 1 | 0.5275 | 1.18075 | 0.9895 | 0.52675 | **Virus Control** | **Virus Control+ 10% DMSO** | **No Virus** | **50% Control** |
| 0.3 | 0.502 | 0.58525 | 0.50925 | 0.50925 | 0.5091 | 0.511 | 1.84955 | 1.1798 |
| 0.1 | 0.50475 | 0.53475 | 0.49825 | 0.51175 | | | | |
| 0.03 | 0.4915 | 0.51425 | 0.49275 | 0.50125 | | | | |
| 0.01 | 0.48 | 0.50275 | 0.4895 | 0.5085 | | | | |
| 0.003 | | 0.4875 | 0.48825 | 0.50175 | | | | |

| | | | | | | | | |
|---|---|---|---|---|---|---|---|---|
| nd= not determined | | | | | | | | |

**Table 4**

| **Compound** | **IC**_{**50**}**mM** |
|---|---|
| | |
| 1 | 1.0 |
| 2 | ~1.0 |
| 4 | 1.9 |
| 3 | >1.0 |

The foregoing description details specific compounds, compositions, methods and uses which can be employed to practice the present invention.

## Claims

1. Use of a poly-maleimide derivative in the manufacture of a medicament for the prophylaxis and/or treatment of microbial infection which affect the reproductive system wherein the maleimide compound is a compound of general formula (I): 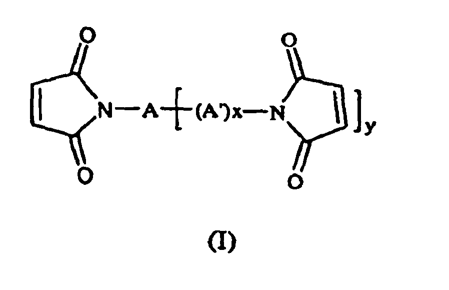 wherein
A is represented by (R¹)ₙ-(R²)ₚ-(R³)_{q}, wherein each of R¹, R² and R³ is independently selected from:
an acyclic aliphatic group having from 1-20 carbon atoms;
a polyalkylene glycol group having an average molecular weight in the range 100 to 1000;
a cyclic aliphatic group having from 4-20 carbon atoms;
a monocyclic or polycyclic aromatic hydrocarbon group having from 6-18 ring carbon atoms;
a non-aromatic heterocyclic group having from 3 to 8 ring atoms, or
a monocyclic or polycyclic aromatic heterocyclic group having from 5 to 20 ring atoms;
and n, p and q each represent 0 or 1 such that the sum of n+p+q = 1-3;
or A substituted by one or more groups independently selected from: 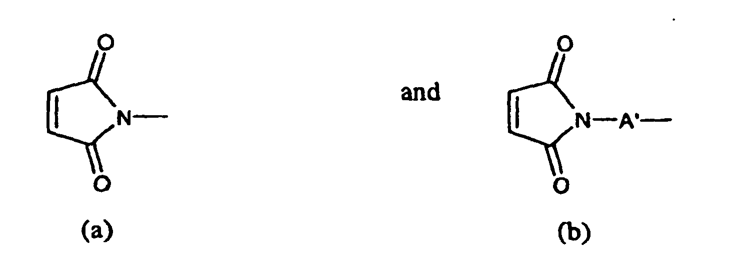
wherein A', which may be the same as or different from A, represents a group (R¹)ₙ-(R²)ₚ-(R³)_{q}, wherein R¹, R², R³, n, p and q are as defined above and wherein X is 0 or 1 and y is 1 to 6;
or a salt, or ester thereof.

2. Use according to claim 1 wherein the infection is a bacterial, fungal or protozoal infection.

3. Use according to claim 1 wherein the infection is a viral infection.

4. Use according to claim 3 wherein the viral infection is caused by a retrovirus, such as HIV.

5. Use according to any of claims 1 to 4 wherein the infection is a sexually transmitted infection.

6. Use according to claims 1 to 5 wherein the maleimide is a compound of formula (II): 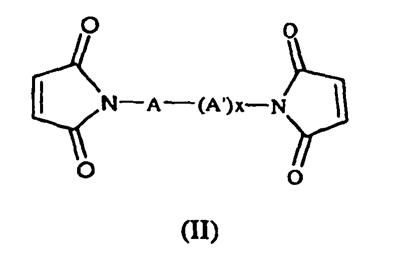 wherein A, A¹ and x are as defined in claim 1.

7. Pharmaceutical formulation comprising a maleimide derivative as defined in claim 1 or 6.

8. A compound of formula (V) 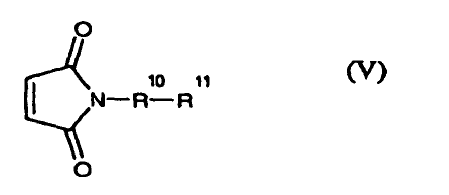 wherein R¹¹ is 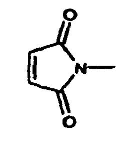 and R¹⁰ is a group R²⁰-R²¹-R²²
wherein R²⁰ is aryl;
R²¹ is -(OCH₂CH₂)ₙ wherein n is 3 to 15;
R²² is a group O-R²³ wherein R²³ is C₁₋₁₀ alkyl;
or R²¹ is -(OCH₂CH₂)ₙ wherein n is 15;
R²² is a group O-R²³ wherein R²³ is aryl.

9. A compound of formula (V) 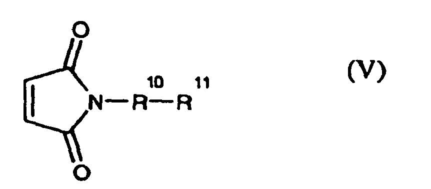 wherein R¹¹ is 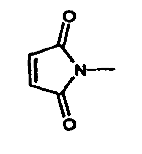 and R¹⁰ is C₂₋₁₀ alkyl, or a group R²⁰-R²¹-R²²
wherein R²⁰ is aryl;
R²¹ is -(OCH₂CH₂)ₙ- wherein n is 3 to 15;
R²² is a group O-R²³ wherein R²³ is aryl or C₁₋₁₀ alkyl,
for use in medicine.

10. A compound as claimed in claim 9 selected from

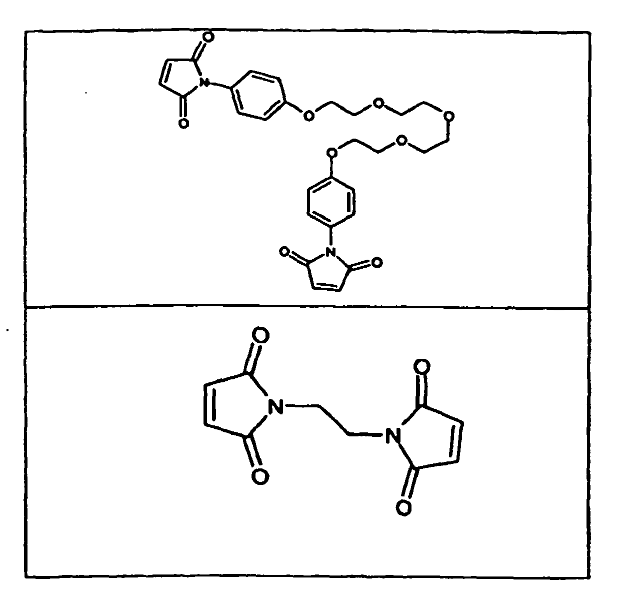

11. A compound as claimed in claim 9 or claim 10 for preventing or treating a microbial infection which affects the reproductive system.

## Patentansprüche

1. Verwendung eines Polymaleimid-Derivats bei der Herstellung eines Arzneimittels für die Prophylaxe und/oder Behandlung einer mikrobiellen Infektion, die das reproduktive (Fortpflanzungs-)System beeinflusst, wobei es sich bei der Maleimid-Verbindung um eine Verbindung der allgemeinen Formel (I) handelt: 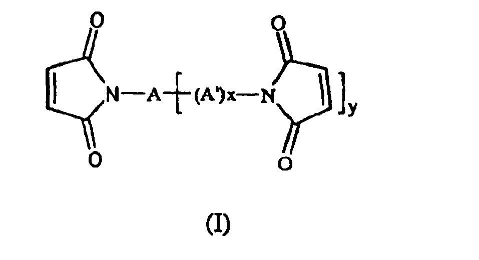 worin bedeuten:
A (R¹)ₙ-(R²)ₚ-(R³)_{q}, worin jeder der Reste R¹, R² und R³ unabhängig voneinander ausgewählt ist aus
einer acyclischen aliphatischen Gruppe mit 1 bis 20 Kohlenstoffatomen; einer Polyalkylenglycol-Gruppe mit einem durchschnittlichen Molekulargewicht in dem Bereich von 100 bis 1000;
einer cyclischen aliphatischen Gruppe mit 4 bis 20 Kohlenstoffatomen;
einer monocyclischen oder polycyclischen aromatischen Kohlenwasserstoff-Gruppe mit 6 bis 18 Ring-Kohlenstoffatomen;
einer nicht-aromatischen heterocyclischen Gruppe mit 3 bis 8 Ringatomen, oder
einer monocyclischen oder polycyclischen aromatischen heterocyclischen Gruppe mit 5 bis 20 Ringatomen; und
n, p und q stehen für die Zahl 0 oder 1, mit der Maßgabe, dass die Summe von n + p + q = 1 bis 3; oder
worin A substituiert ist durch eine oder mehrere Gruppen, die unabhängig voneinander ausgewählt sind aus 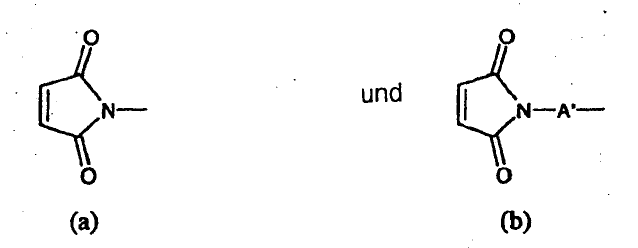 worin A', das mit A identisch oder davon verschieden sein kann, steht für eine Gruppe (R¹)ₙ-(R²)ₚ- (R³)_{q}, worin R¹, R², R³, n, p und q die oben angegebenen Bedeutungen haben und
x steht für 0 oder 1 und y steht für eine Zahl von 1 bis 6;
oder ein Salz oder einen Ester davon.

2. Verwendung nach Anspruch 1, bei der die Infektion eine durch Bakterien, Fungi oder Protozoen verursachte Infektion ist.

3. Verwendung nach Anspruch 1, bei der die Infektion eine Virus-Infektion ist.

4. Verwendung nach Anspruch 3, bei der die Virus-Infektion durch ein Retrovirus, wie z.B. HIV, hervorgerufen worden ist.

5. Verwendung nach einem der Ansprüche 1 bis 4, bei der die Infektion eine durch Geschlechtsverkehr übertragene Infektion ist.

6. Verwendung nach den Ansprüchen 1 bis 5, bei der das Maleimid eine Verbindung der Formel (II) ist: 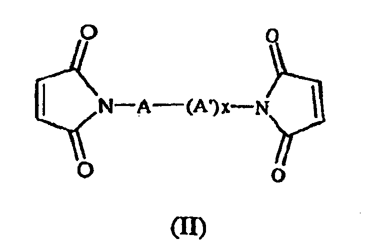 worin A, A' und x die in Anspruch 1 angegebenen Bedeutungen haben.

7. Pharmazeutische Formulierung, die ein Maleimid-Derivat umfasst, wie es in Anspruch 1 oder 6 definiert ist.

8. Verbindung der Formel (V) 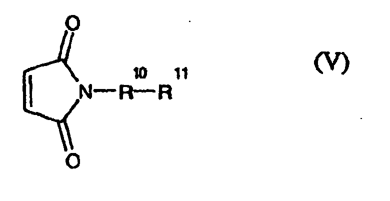 worin R¹¹ steht für 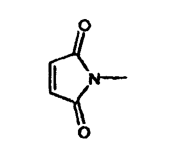 und R¹⁰ steht für eine Gruppe R²⁰-R²¹-R²²,
worin bedeuten:
R²⁰ Aryl;
R²¹ -(OCH₂CH₂)ₙ-, worin n steht für eine Zahl von 3 bis 15; und
R²² eine Gruppe O-R²³, worin R²³ ein C₁₋₁₀-Alkyl darstellt; oder
R²¹ -(OCH₂CH₂)ₙ, worin n für die Zahl 15 steht; und
R²² eine Gruppe O-R²³, worin R²³ für Aryl steht.

9. Verbindung der Formel (V) 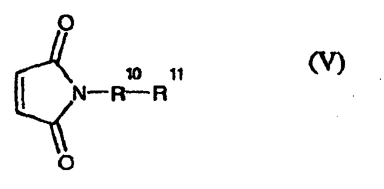 worin R¹¹ steht für 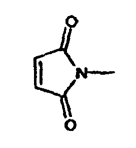 und
R¹⁰ steht für C₂₋₁₀-Alkyl oder eine Gruppe R²⁰-R²¹-R²², worin bedeuten:
R²⁰ Aryl;
R²¹ -(OCH₂CH₂)ₙ-, worin n steht für eine Zahl von 3 bis 15; und
R²² eine Gruppe O-R²³, worin R²³ für Aryl oder C₁₋₁₀-Alkyl steht,
für die Verwendung in der Medizin.

10. Verbindung nach Anspruch 9, ausgewählt aus

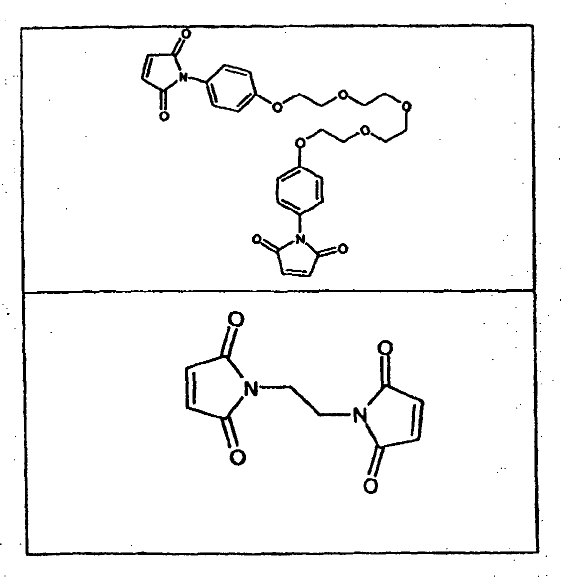

11. Verbindung nach Anspruch 9 oder 10 zur Prävention oder Behandlung einer mikrobiellen Infektion, die das reproduktive (Fortpflanzungs-)System beeinflusst.

## Revendications

1. Utilisation d'un dérivé de polymaléimide dans la fabrication d'un médicament pour la prophylaxie et/ou le traitement d'une infection microbienne qui affecte l'appareil reproducteur, dans laquelle le composé de maléimide est un composé de formule (I) générale : 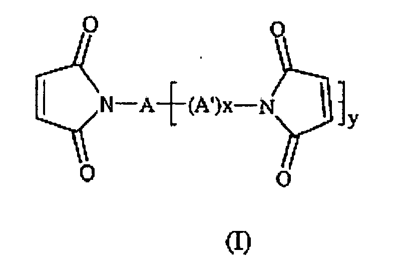 dans laquelle :
A est représenté par (R¹)ₙ-(R²)ₚ-(R³)_{q}, dans laquelle chacun des groupements R¹, R² et R³ est choisi indépendamment parmi :
un groupement aliphatique acyclique ayant 1 à 20 atomes de carbone;
un groupement polyalkylèneglycol ayant un poids moléculaire moyen dans la plage de 100 à 1000;
un groupement aliphatique cyclique ayant 4 à 20 atomes de carbone;
un groupement hydrocarboné aromatique monocyclique ou polycyclique ayant 6 à 18 atomes de carbone sur le cycle;
un groupement hétérocyclique non aromatique ayant 3 à 8 atomes sur le cycle, ou
un groupement hétérocyclique aromatique monocyclique ou polycyclique ayant 5 à 20 atomes sur le cycle;
et n, p et q représentent chacun 0 ou 1 de sorte que la somme n + p + q = 1 à 3;
ou A substitué par un ou plusieurs groupements choisis indépendamment parmi : 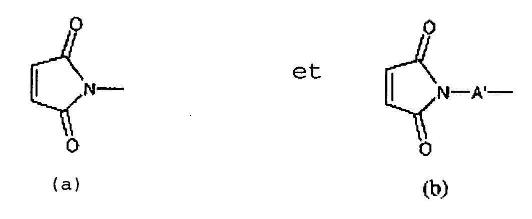 dans lesquelles A', qui peut être identique ou différent de A, représente un groupement (R¹)ₙ-(R²)ₚ-(R³)_{q}, dans lequel R¹, R² et R³, n, p et q sont comme défini ci-dessus et où X est 0 ou 1 et y est 1 à 6;
ou d'un de ses sels ou esters.

2. Utilisation selon la revendication 1, dans laquelle l'infection est une infection bactérienne, fongique ou protozoaire.

3. Utilisation selon la revendication 1, dans laquelle l'infection est une infection virale.

4. Utilisation selon la revendication 3, dans laquelle l'infection virale est provoquée par un rétrovirus tel que le VIH.

5. Utilisation selon l'une quelconque des revendications 1 à 4, dans laquelle l'infection est une infection transmise sexuellement.

6. Utilisation selon les revendications 1 à 5, dans laquelle le maléimide est un composé de formule (II) : 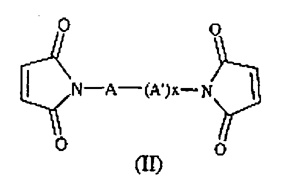 dans laquelle A, A' et x sont comme défini dans la revendication 1.

7. Formulation pharmaceutique comprenant un dérivé de maléimide comme défini dans la revendication 1 ou 6.

8. Composé de formule (V) : 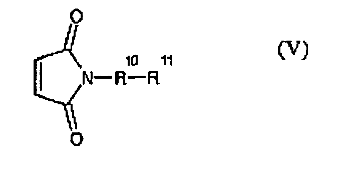 dans laquelle R¹¹ est : 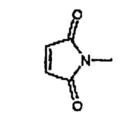 et R¹⁰ est un groupement R²⁰-R²¹-R²²,
dans lequel :
R²⁰ est un aryle;
R²¹ est -(OCH₂CH₂)ₙ- où n est 3 à 15;
R²² est un groupement O-R²³ où R²³ est un alkyle en C₁₋₁₀;
ou R²¹ est -(OCH₂CH₂)ₙ- où n est 15;
R²² est un groupement O-R²³ où R²³ est un aryle.

9. Composé de formule (V) : 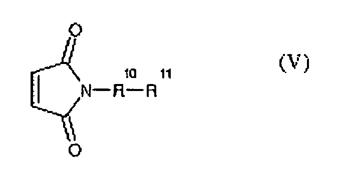 dans laquelle R¹¹ est : 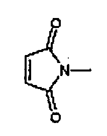 et R¹⁰ est un alkyle en C₂-C₁₀ ou un groupement R²⁰-R²¹⁻R²²,
dans lequel :
R²⁰ est un aryle;
R²¹ est -(OCH₂CH₂)ₙ- où n est 3 à 15;
R²² est un groupement O-R²³ où R²³ est un aryle
ou un alkyle en C₁₋₁₀,
pour usage en médecine.

10. Composé selon la revendication 9,choisi parmi :

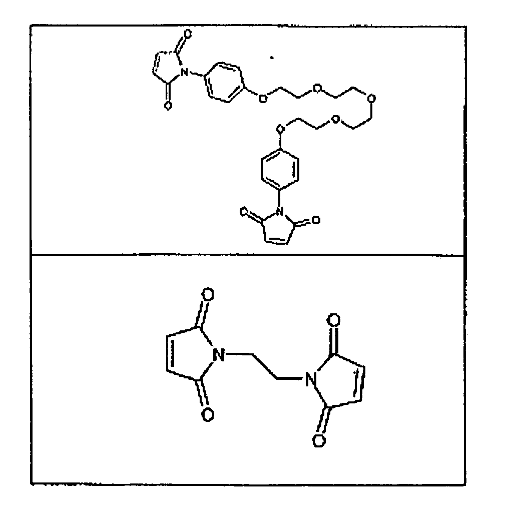

11. Composé selon la revendication 9 ou 10, pour empêcher ou traiter une infection microbienne qui affecte l'appareil reproducteur.
